# EUROPÄISCHE PATENTSCHRIFT

(11) **EP 3 375 864 B1**
(45) Veröffentlichungstag und Bekanntmachung des Hinweises auf die Patenterteilung: **09.09.2020**
(21) Anmeldenummer: 18000171.1
(22) Anmeldetag: 20.02.2018
(51) Int. Cl.: C12N 5/00

(54) **VERFAHREN ZUR DREIDIMENSIONALEN KULTIVIERUNG VON ZELLEN IN EINEM FLÜSSIGEN NÄHRMEDIUM**
METHOD FOR THREE-DIMENSIONAL CULTURING OF CELLS IN A LIQUID NUTRIENT MEDIUM
PROCÉDÉ DE CULTURE TRIDIMENSIONNELLE DE CELLULES DANS UN MILIEU NUTRITIF LIQUIDE

(30) Priorität: 13.03.2017 DE 102017002438
(43) Veröffentlichungstag der Anmeldung: 19.09.2018
(73) Patentinhaber: Karlsruher Institut für Technologie, 76131 Karlsruhe (DE)
(72) Erfinder: LEE-THEDIECK, Cornelia, 76297 Stutensee (DE); RÖDLING, Lisa, 4054 Basel (CH); FRANZREB, Matthias, 76185 Karlsruhe (DE); RAIC, Annamarija, 76137 Karlsruhe (DE); VOLZ, Esther, 2512 BK Den Haag (NL); RAPP, Bastian, 76135 Karlsruhe (DE)
(74) Vertreter: Müller-Boré & Partner Patentanwälte PartG mbB

(56) Entgegenhaltungen:
- WO-A1-2010/036957
- US-A1- 2014 302 110
- DE SANTIS ROBERTO ET AL: "3D fibre deposition and stereolithography techniques for the design of multifunctional nanocomposite magnetic scaffolds", JOURNAL OF MATERIALS SCIENCE: MATERIALS IN MEDICINE, SPRINGER NEW YORK LLC, UNITED STATES, Bd. 26, Nr. 10, 29. September 2015 (2015-09-29), Seiten 1-9, XP035550804, ISSN: 0957-4530, DOI: 10.1007/S10856-015-5582-4 [gefunden am 2015-09-29]
- GLAUCO R. SOUZA ET AL: "Three-dimensional tissue culture based on magnetic cell levitation", NATURE NANOTECHNOLOGY, Bd. 5, Nr. 4, 14. März 2010 (2010-03-14), Seiten 291-296, XP055233171, GB ISSN: 1748-3387, DOI: 10.1038/nnano.2010.23
- LEE EUNJEE A ET AL: "Application of magnetic nanoparticle for controlled tissue assembly and tissue engineering", ARCHIVES OF PHARMACAL RESEARCH, NATL. FISHERIES UNIVERSITY, PUSAN, KR, Bd. 37, Nr. 1, 6. Dezember 2013 (2013-12-06), Seiten 120-128, XP035312367, ISSN: 0253-6269, DOI: 10.1007/S12272-013-0303-3 [gefunden am 2013-12-06]
- NIMA A. JALILI ET AL: "Nanoengineered thermoresponsive magnetic hydrogels for biomedical applications : Jalili et al.", BIOENGINEERING & TRANSLATIONAL MEDICINE, Bd. 1, Nr. 3, 1. September 2016 (2016-09-01), Seiten 297-305, XP055471184, ISSN: 2380-6761, DOI: 10.1002/btm2.10034
- YUHUI LI ET AL: "Magnetically actuated cell-laden microscale hydrogels for probing strain-induced cell responses in three dimensions", NPG ASIA MATERIALS, Bd. 8, Nr. 1, 1. Januar 2016 (2016-01-01), Seiten e238-e238, XP055471188, JP ISSN: 1884-4049, DOI: 10.1038/am.2015.148
- RAIC ANNAMARIJA ET AL: "Biomimetic macroporous PEG hydrogels as 3D scaffolds for the multiplication of human hematopoietic stem and progenitor cells", BIOMATERIALS, Bd. 35, Nr. 3, 28. Oktober 2013 (2013-10-28), Seiten 929-940, XP028773641, ISSN: 0142-9612, DOI: 10.1016/J.BIOMATERIALS.2013.10.038
- LISA RÖDLING ET AL: "Magnetic Macroporous Hydrogels as a Novel Approach for Perfused Stem Cell Culture in 3D Scaffolds via Contactless Motion Control", ADVANCED HEALTHCARE MATERIALS, 19. Januar 2018 (2018-01-19), Seite 1701403, XP055470923, DE ISSN: 2192-2640, DOI: 10.1002/adhm.201701403

## Beschreibung

Die vorliegende Erfindung betrifft ein Verfahren zur dreidimensionalen Kultivierung von Zellen in einem flüssigen Nährmedium unter Verwendung eines Trägers mit magnetischen Eigenschaften. Ferner betrifft die vorliegende Erfindung die Verwendung eines solchen Trägers zur dreidimensionalen Kultivierung von Zellen in einem flüssigen Nährmedium.

In der Gewebekultur werden Zellen heutzutage nach wie vor hauptsächlich in zweidimensionalen Strukturen, beispielsweise auf flachem Zellkulturplastik, gezüchtet, was für die Zellen, die ursprünglich aus einer dreidimensionalen Umgebung innerhalb eines Gewebes stammen, eine künstliche Situation darstellt. Dies kann unter Umständen zu Artefakten führen, welche durch die unnatürliche Art der Kultivierung hervorgerufen werden.

Aus diesem Grund sind verschiedene Verfahren und Materialien entwickelt worden, die der Kultivierung von Zellen in einer dreidimensionalen Umgebung dienen. Grundsätzlich haben all diese Ansätze gemein, dass innerhalb einer dreidimensionalen Struktur die Versorgung der Zellen mit Nährstoffen nur in begrenztem Umfang mittels Diffusion möglich ist, d.h. lediglich bis zu einer Entfernung von etwa 1 mm.

Im Stand der Technik sind verschiedene Ansätze beschrieben, welche einen über die Diffusion hinausgehenden Stoffaustausch ermöglichen sollen. Üblicherweise geschieht dies mit Hilfe von Perfusionsreaktoren, in denen die Gerüststrukturen, welche die Zellen enthalten, mit der Nährlösung durch-, über- oder unterströmt werden. Auch kann der Austausch der Nährlösung durch mechanische Bewegung der Gerüststrukturen im wässrigen Medium, beispielsweise in Rührkulturen oder in Rollerflaschen, begünstigt werden.

Die erste Herangehensweise, d.h. der Einsatz von Perfusionsreaktoren, hat allerdings den Nachteil, dass eine Parallelisierung der Zellkultivierung über den Labormaßstab hinaus kaum zu realisieren ist. Da die Perfusion mittels Pumpensysteme den Einsatz spezieller Reaktorkapseln erfordert, können hierfür in der Regel auch keine handelsüblichen Einwegzellkulturmaterialien verwendet werden.

In diesem Zusammenhang beschreiben A. Raic et al. (Biomaterials 2014, 35, 929-940) biomimetische makroporöse PEG-Hydrogele als dreidimensionale Gerüste für die Vermehrung von humanen hämatopoetischen Stamm- und Progenitorzellen.

Die zweite Herangehensweise, d.h. die mechanische Bewegung der Trägermaterialien in der Nährlösung, führt zu einer mechanischen Beanspruchung der Zellträger aufgrund von Kollisionen mit dem Zellkulturgefäß und anderen Trägern, wodurch das Risiko einer Beschädigung des Trägermaterials besteht. Ferner lassen sich beim mechanischen Rühren der Nährlösung oder beim Schütteln der Zellkulturgefäße die Positionen der Träger und auch deren Bewegung einschließlich ihrer Geschwindigkeit kaum exakt kontrollieren.

Der vorliegenden Erfindung liegt somit die Aufgabe zugrunde ein Verfahren bereitzustellen, welches die dreidimensionale Kultivierung von Zellen in einem flüssigen Nährmedium erlaubt, wobei ein über die Diffusion hinausgehender Stoffaustausch auf apparativ einfache Art und Weise sowie ohne mechanische Beanspruchung des die Zellen enthaltenden Trägers bei exakter Positionierung und Bewegung des Trägers realisiert sein soll.

Diese Aufgabe wird durch die in den Ansprüchen gekennzeichneten Ausführungsformen der vorliegenden Erfindung gelöst.

Insbesondere wird erfindungsgemäß ein Verfahren zur dreidimensionalen Kultivierung von Zellen in einem flüssigen Nährmedium bereitgestellt, wobei sich die Zellen in den Poren eines Trägers mit magnetischen Eigenschaften befinden. Das erfindungsgemäße Verfahren ist dadurch gekennzeichnet, dass der Träger durch ein externes Magnetfeld innerhalb des flüssigen Nährmediums bewegt wird und der Träger ein makroporöses Polymernetzwerk und magnetische Nanopartikel, welche kovalent oder nicht-kovalent an das Polymernetzwerk gebunden sind, umfasst, wobei die Porenräume des Trägers in offenem Kontakt zueinander stehen, so dass ein Stoffaustausch mit dem flüssigen Nährmedium stattfindet.

Durch den Einsatz eines porösen Trägers mit magnetischen Eigenschaften in Verbindung mit einem externen Magnetfeld, das den Träger in der Nährlösung bewegt, wird bei der dreidimensionalen Kultivierung von Zellen in einem flüssigen Nährmedium ein über die Diffusion hinausgehender Stoffaustausch erreicht, wobei die im Stand der Technik bekannten Nachteile überwunden werden. Insbesondere erlaubt das erfindungsgemäße Verfahren eine exakte Kontrolle der Bewegung des die Zellen enthaltenden Trägers, ohne diesen mechanisch zu beanspruchen. Ferner erfordert das erfindungsgemäße Verfahren lediglich einen vergleichsweise geringen apparativen Aufwand und lässt sich problemlos parallelisieren.

Mit dem erfindungsgemäßen Verfahren lassen sich Zellen in einer dreidimensionalen Umgebung kultivieren. Im Gegensatz zu zweidimensionalen Zellkulturen spiegeln dreidimensionale Zellkulturen die räumliche Ausdehnung des Gewebes wider. Dreidimensionale Zellkulturen eignen sich u.a. als Modellsysteme zur Studie von Phänomenen wie beispielsweise Zellmigration, -differenzierung, -überleben und -wachstum. Zusätzlich ermöglicht die dreidimensionale Kultivierung eine natürliche Zell-Zell-Kommunikation über kurze Distanzen durch auto- und parakrine Signale, welche in zweidimensionalen Zellkulturen aufgrund der schnellen Verdünnung der Signale in einem großen Volumen in der Regel nicht möglich ist. Darüber hinaus ist die dreidimensionale Kultivierung wichtig zur Entwicklung von Gewebemodellen, welche beispielsweise zur *in vitro*-Testung von Medikamenten verwendet werden können.

Die vorliegende Erfindung ist nicht auf bestimmte Zelltypen beschränkt. Insbesondere kann in dem vorstehend definierten Verfahren jede Art von Zellen in Betracht gezogen werden, für die eine dreidimensionale Kultivierung von Interesse ist. Bei den im erfindungsgemäßen Verfahren zum Einsatz kommenden Zellen, die in einer dreidimensionalen Umgebung kultiviert werden, kann es sich sowohl um Suspensionsals auch um adhärent wachsende Zellen handeln. Nicht einschränkend sind als in Frage kommende Zelltypen prokaryotische Zellen einschließlich Bakterien und Archaeen, sowie eukaryotische Zellen einschließlich Hefezellen (wie *Saccharomyces,* z.B. *Saccharomyces cerevisiae*), Insektenzellen (z.B. *Drosophila melanogaster*), Pflanzenzellen und Säugerzellen (z.B. HEK 293, HeLa, CHO, hämatopoetische Stamm- und Progenitorzellen) genannt, wobei die Säugerzellen auch humane primäre Zellen und Zelllinien einschließen. In einer Ausführungsform des erfindungsgemäßen Verfahrens werden Säugerzellen, beispielsweise Stammzellen, der dreidimensionalen Kultivierung unterzogen, wobei es sich vorzugsweise nicht um embryonale Stammzellen handelt. Exemplarisch sind hier als Zellen für das erfindungsgemäße Verfahren humane hämatopoetische Stamm- und Progenitorzellen sowie humane mesenchymale Stamm-/Stromazellen genannt.

In gleicher Weise unterliegt das flüssige Nährmedium, allgemein auch als Nährlösung bezeichnet, erfindungsgemäß keinen Einschränkungen. Entsprechend des zu kultivierenden Zelltyps wird der Fachmann ein flüssiges Nährmedium, üblicherweise auf wässriger Basis, mit geeigneter Zusammensetzung auswählen. Im erfindungsgemäßen Verfahren wird das flüssige Nährmedium typischerweise in einem handelsüblichen Kulturgefäß bereitgestellt.

Die mit dem erfindungsgemäßen Verfahren zu kultivierenden Zellen befinden sich in den Poren eines Trägers. Die Zellen nehmen den Träger mit seiner makroporösen Morphologie als dreidimensionale Gerüststruktur wahr, wodurch sie in einer vergleichsweise natürlichen Umgebung wachsen können. Die Porenräume des Trägers stehen in offenem Kontakt zueinander, d.h. sie sind in Bezug aufeinander und die Umgebung permeabel, so dass ein Stoffaustausch mit dem flüssigen Nährmedium stattfindet. Unter Stoffaustausch ist hier gleichermaßen der Eintrag als auch der Austrag von Stoffen in den bzw. aus dem Träger gemeint, mit dem Ziel einen Konzentrationsausgleich zwischen dem Inneren des Trägers und dem flüssigen Nährmedium herzustellen.

Abgesehen von der vorstehend beschriebenen Porosität, welche die Besiedlung mit Zellen sowie den Stoffaustausch mit dem flüssigen Nährmedium gewährleistet, weist der Träger gemäß der vorliegenden Erfindung magnetische Eigenschaften auf. Im erfindungsgemäßen Verfahren wird der Träger so durch ein externes Magnetfeld bewegt.

Die magnetischen Eigenschaften des Trägers, welcher synonym auch als magnetischer Träger bezeichnet werden kann, sind nicht auf eine bestimmte Art von Magnetismus beschränkt. Die magnetischen Eigenschaften des Trägers können beispielsweise auf Ferromagnetismus, Paramagnetismus und/oder Superparamagnetismus beruhen. Einzige Voraussetzung ist hierbei lediglich, dass der Träger auf die Anwesenheit eines externen Magnetfeldes reagiert, indem er beispielsweise einer räumlichen Änderung des externen Magnetfeldes mit einer entsprechenden räumlichen Änderung seinerseits folgt.

Das erfindungsgemäße Verfahren erlaubt durch das Einwirken des externen Magnetfeldes auf den Träger dessen gezielte Bewegung in dem flüssigen Nährmedium. Durch eine gezielte Bewegung des Trägers wird der Austausch an löslichen Stoffen, beispielsweise an Nährstoffen wie Glukose, Gase, Salze, Wachstumsfaktoren, etc. innerhalb der Poren des Trägers über die Diffusion hinaus unterstützt, was einer eventuellen Verarmung an Nährstoffen oder einer Anreicherung von für das Zellwachstum inhibierenden Faktoren entgegenwirkt.

Da die Bewegung des Trägers erfindungsgemäß durch ein externes Magnetfeld herbeigeführt wird, können handelsübliche Einwegzellkulturmaterialien verwendet werden. Beispielsweise können hier Einwegzellkulturmaterialien in Form eines Röhrchens zum Einsatz kommen, welche aufgrund ihrer Röhrenform von einem Ringmagneten umschlossen werden können, der so im Inneren des Röhrchens ein Magnetfeld erzeugt. Derartige kommerziell erhältliche Einwegzellkulturmaterialien sind beispielsweise Cellstar® Tubes, Falcon® Tubes, Fisherbrand™ Tubes und Pyrex™ Tubes, wobei diese prinzipiell aus Kunststoff oder aus Glas gefertigt sein können. Ferner ist es möglich Zellkulturgefäße in Form von Platten mit Vertiefungen, welche auch als Wellplates bezeichnet werden, zu verwenden. Der Träger kann dann in den Vertiefungen, welche mit dem flüssigen Nährmedium gefüllt sind, platziert werden. Die Bewegung des Trägers kann in diesem Fall durch Magneten, welche der Platte von oben und/oder von unten angenähert werden, erreicht werden. Wellplates für die Zellkultur sind kommerziell von verschiedenen Anbietern, beispielsweise von Greiner Bio-One, Sarstedt oder Nunc, und in verschiedenen Formaten, beispielsweise als 6-, 12-, 24-, 48- oder 96-well-Format, mit normaler well-Tiefe oder besonders großer weil-Tiefe (deep well) erhältlich.

Ferner erlaubt die Bewegung des Trägers durch das externe Magnetfeld eine Parallelisierung der dreidimensionalen Zellkultivierung, d.h. das erfindungsgemäße Verfahren eignet sich auch zur Anwendung über den Labormaßstab hinaus.

Die Bewegung des die Zellen enthaltenden Trägers kann ferner berührungsfrei erfolgen. Der die Bewegung hervorrufende Aktor befindet sich demnach außerhalb des flüssigen Nährmediums, d.h. der Träger wird nicht durch direkten Kontakt bzw. durch Anströmung beeinflusst. Insbesondere bleibt hierdurch eine mechanische Beanspruchung des Trägers aus, da sich eine Kollision mit dem Kulturgefäß oder mit anderen Trägern innerhalb des flüssigen Nährmediums vermeiden lässt. Auf diese Weise ist eine besonders schonende Kultivierung der Zellen in einer dreidimensionalen Umgebung möglich.

Das externe Magnetfeld kann grundsätzlich auf beliebigem Weg bereitgestellt werden. In einer Ausführungsform des erfindungsgemäßen Verfahrens wird das externe Magnetfeld durch einen sich außerhalb des flüssigen Nährmediums befindlichen Magneten hervorgerufen. Bei Letzterem kann es sich um einen handelsüblichen Dauermagneten handeln, dessen Form keiner besonderen Einschränkung unterliegt. In einer bevorzugten Ausführungsform kommt ein Ringmagnet zum Einsatz, welcher ein röhrenförmiges Kulturgefäß und das darin enthaltene flüssige Nährmedium umgibt. Anschaulich umschließt der Ringmagnet das flüssige Nährmedium, welches sich typischerweise in dem röhrenförmigen Kulturgefäß befindet, gewissermaßen wie eine Schlaufe. Wird hingegen eine Wellplate als Kulturgefäß verwendet, befinden sich die Magneten ober- und/oder unterhalb hiervon und können auf diese Weise von oben und/oder von unten angenähert werden.

Wird im erfindungsgemäßen Verfahren das externe Magnetfeld durch einen Magneten bzw. Dauermagneten hervorgerufen, wird das externe Magnetfeld vorzugsweise durch Positionsänderung des Magneten in Bezug auf das flüssige Nährmedium räumlich variiert. Der sich im flüssigen Nährmedium befindliche Träger folgt sodann der geänderten Position des Magneten, d.h. der Träger passt seine eigene Position der geänderten Position des externen Magnetfeldes an. Auf diese Weise lässt sich der Träger im flüssigen Nährmedium kontrolliert bewegen, so dass ein über die Diffusion hinausgehender Stoffaustausch gewährleistet ist.

In einer weiteren Ausführungsform des erfindungsgemäßen Verfahrens wird durch räumliche Variation des externen Magnetfeldes, bedingt durch die Positionsänderung des Magneten, der Träger entlang einer vorgegebenen Raumrichtung hin und her bewegt. Der das externe Magnetfeld hervorrufende Magnet, beispielsweise ein Ringmagnet, lässt sich etwa durch eine elektrisch betriebene Vorrichtung periodisch räumlich verfahren, wodurch sich die dreidimensionale Kultivierung der Zellen im erfindungsgemäßen Verfahren problemlos automatisieren lässt. Die periodische Bewegung des Magneten und damit auch die des Trägers entlang einer vorgegebenen Raumrichtung sind hier nur beispielhaft genannt. Prinzipiell lässt sich das externe Magnetfeld auch auf andere Weise periodisch ändern und so eine Automatisierung erreichen. Auch ist ein nicht-periodisches räumliches Verfahren des Magneten prinzipiell denkbar. Allerdings ist ein eindimensionales Verfahren des Magneten entlang einer bestimmten Raumrichtung, wobei der Magnet periodisch in Vorwärts- und Rückwärtsrichtung bewegt wird, vergleichsweise ohne größeren apparativen Aufwand umsetzbar. Dies gilt insbesondere bei der Verwendung eines das flüssige Nährmedium umgebenden Ringmagneten, der vertikal, d.h. nach oben und unten, verfahren wird.

In einer weiteren Ausführungsform wird das externe Magnetfeld elektromagnetisch hervorgerufen. Durch eine zeitlich variierende Stromstärke in einer Anordnung von mindestens zwei Elektromagnetspulen lässt sich das externe Magnetfeld beispielsweise räumlich variieren.

Der im erfindungsgemäßen Verfahren zum Einsatz kommende Träger ist zur dreidimensionalen Kultivierung von Zellen geeignet, wobei die Porosität des Trägers notwendigerweise dergestalt ist, dass die Zellen die miteinander verbundenen Poren des Trägers besiedeln können und ein Stoffaustausch mit dem flüssigen Nährmedium möglich ist. Weiterhin weist der Träger notwendigerweise magnetische Eigenschaften auf, um durch das externe Magnetfeld bzw. durch dessen Änderung bewegt zu werden. Ansonsten unterliegt der Träger im erfindungsgemäßen Verfahren keinen weiteren Einschränkungen.

Gemäß der vorliegenden Erfindung umfasst der Träger ein makroporöses Polymernetzwerk. Das Einbringen von Makroporen in die polymere Netzwerkstruktur des Trägers gelingt beispielsweise durch Auswaschen (Leaching) von zuvor einpolymerisiertenSalzkristallen oder anderen mikroskaligen Festkörpern. Allerdings kann hierzu auch jedes andere dem Fachmann bekannte Verfahren eingesetzt werden, darunter beispielsweise auch die Cryogelierung. Die Makroporosität in der polymeren Netzwerkstruktur gewährleistet die für die Ansiedlung der Zellen notwendige Porosität. Die Porengröße kann u.a. durch Wahl des Porogens, insbesondere seiner Größe, sowie der Herstellungsbedingungen an die Erfordernisse des jeweiligen Zelltyps angepasst werden.

Durch Variation der Vernetzungsdichte bei der Synthese des Polymernetzwerkes kann ferner die für einen bestimmten Zelltyp erforderliche Steifigkeit in der polymeren Netzwerkstruktur eingestellt werden. Durch geeignete Wahl der Monomere ist das Polymernetzwerk ferner imstande Wassermoleküle aufzunehmen, weswegen es auch als Hydrogel aufgefasst werden kann. Hierdurch wird eine für die Zellen möglichst natürliche Umgebung geschaffen. Zu diesem Zweck kann das Polymernetzwerk beispielsweise Einheiten aus Poly(ethylenglykol) (PEG), Poly-L-Lysin (PLL), Poly(2-hydroxyethylmethacrylat) (PHEMA), Polyacrylamid, Hyaluronsäure oder Proteine wie Collagen umfassen.

Neben dem Polymernetzwerk umfasst der Träger noch magnetische Nanopartikel, welche dem Träger die für das erfindungsgemäße Verfahren notwendigen magnetischen Eigenschaften verleihen. Ohne hierauf beschränkt zu sein, können beispielsweise superparamagnetische Nanopartikel eingesetzt werden.

Die Zusammensetzung und die Größe der magnetischen Nanopartikel unterliegen erfindungsgemäß keinen besonderen Einschränkungen. Typischerweise sind die magnetischen Nanopartikel eisenhaltig. Es kann sich beispielweise um Eisenoxidnanopartikel aus Magnetit handeln. Die Abmessungen der magnetischen Nanopartikel, worunter ihr gemittelter Teilchendurchmesser zu verstehen ist, liegen beispielsweise in einem Bereich von 5 bis 200 nm, wobei ein Bereich von 10 bis 100 nm bevorzugt ist. Die magnetischen Nanopartikel sind kovalent oder nicht-kovalent an das Polymernetzwerk gebunden. Eine kovalente Bindung ist vorteilhaft, da die magnetischen Nanopartikel so mit dem Polymernetzwerk eine stabilere Einheit bilden, wodurch ein eventuelles Herauslösen der magnetischen Nanopartikel aus dem Polymernetzwerk und ein Übergang in das flüssige Nährmedium weitestgehend verhindert werden. Im Falle einer nicht-kovalenten Bindung der magnetischen Nanopartikel an das Polymernetzwerk kann ein solches Herauslösen naturgemäß nicht vollkommen unterbunden werden, da die magnetischen Nanopartikel nur durch vergleichsweise schwache van-der-Waals-Wechselwirkungen in Verbindung mit sterischen Effekten stabilisiert sind.

Eine nicht-kovalente Bindung der magnetischen Nanopartikel an das Polymernetzwerk lässt sich beispielsweise realisieren, indem die magnetischen Nanopartikel in der Gegenwart der das Polymernetzwerk bildenden Monomere einpolymerisiert bzw. eingebunden werden. Hierdurch kommt es u.a. auch zu einem Einschluss der magnetischen Nanopartikel im Inneren des Polymernetzwerkes. Um die magnetischen Nanopartikel kovalent an das Polymernetzwerk zu binden, bedürfen sowohl das Polymernetzwerk als auch die magnetischen Nanopartikel einer geeigneten Funktionalisierung. Dem Fachmann sind hierbei in Frage kommende Funktionalisierungsstrategien sowohl für das Polymernetzwerk als auch für die magnetischen Nanopartikel bekannt, wobei die funktionalisierten magnetischen Nanopartikel geeigneterweise bereits bei der Synthese des Polymernetzwerkes an dieses kovalent gebunden werden. Ebenso wie bei der nicht-kovalenten Bindung wird hierdurch sichergestellt, dass die magnetischen Nanopartikel homogen über das Polymernetzwerk verteilt sind.

Unabhängig von der Bindungssituation in Bezug auf die magnetischen Nanopartikel kann das Polymernetzwerk weiter funktionalisiert sein, um beispielsweise die natürliche Umgebung der Zellen durch bestimmte adhäsive Peptidsequenzen besser nachahmen zu können. Beispiele solcher adhäsiver Peptidsequenzen sind u.a. RGD, IKVAV, PHSRN und LDV, wobei dem Fachmann weitere solcher adhäsiver Peptidsequenzen bekannt sind.

Das in der vorstehenden Ausführungsform beschriebene makroporöse Polymernetzwerk in Verbindung mit den magnetischen Nanopartikeln lässt sich so an den jeweiligen Zelltyp und an die jeweilige Zellumgebung anpassen, wodurch es sich als vergleichsweise besonders natürlicher Träger für die dreidimensionale Kultivierung von Zellen im erfindungsgemäßen Verfahren eignet.

In einem weiteren Aspekt betrifft die vorliegende Erfindung die Verwendung des vorstehend definierten Trägers zur dreidimensionalen Kultivierung von Zellen in einem flüssigen Nährmedium, wobei die erfindungsgemäße Verwendung des Trägers dadurch gekennzeichnet ist, dass dieser durch ein externes Magnetfeld innerhalb des flüssigen Nährmediums bewegt wird und der Träger ein makroporöses Polymernetzwerk und magnetische Nanopartikel, welche kovalent oder nicht-kovalent an das Polymernetzwerk gebunden sind, umfasst, wobei die Porenräume des Trägers in offenem Kontakt zueinander stehen, so dass ein Stoffaustausch mit dem flüssigen Nährmedium stattfindet. Für die erfindungsgemäße Verwendung des Trägers gelten die gleichen Definitionen und Ausführungen, wie sie bereits in Bezug auf das erfindungsgemäße Verfahren genannt und beschrieben worden sind.

Das erfindungsgemäße Verfahren sowie die erfindungsgemäße Verwendung erlauben eine dreidimensionale Kultivierung von Zellen in einem flüssigen Nährmedium. Dabei nehmen die Zellen den Träger, in dessen Poren sie sich befinden, als dreidimensionale Gerüststruktur wahr, wodurch sie in einer möglichst natürlichen dreidimensionalen Umgebung wachsen können.

Durch das externe Magnetfeld in Verbindung mit den magnetischen Eigenschaften des Trägers lässt sich dessen Bewegung einschließlich seiner Geschwindigkeit präzise kontrollieren. Insbesondere wird hierdurch eine mechanische Beanspruchung des Trägers vermieden, wie sie beim Rühren einer Nährlösung oder beim Schütteln eines Zellkulturgefäßes zwangsläufig auftreten würde. Eine Verformung des Trägers kann somit ebenfalls ausgeschlossen werden, da sogar eine berührungsfreie Bewegung gemäß der vorliegenden Erfindung möglich ist.

Die kontrollierte Bewegung des die Zellen enthaltenden Trägers führt zu einem über die Diffusion hinausgehenden Austausch an löslichen Stoffen in dem flüssigen Nährmedium, was insbesondere die Versorgung der Zellen im Trägerinneren mit Nährstoffen oder das Auswaschen inhibierender Faktoren begünstigt.

Ferner erlauben das erfindungsgemäße Verfahren sowie die erfindungsgemäße Verwendung eine Parallelisierung der Zellkultivierung über den Labormaßstab hinaus, wobei es hierbei lediglich eines vergleichsweise geringen apparativen Aufwandes bedarf. Auch lassen sich im Gegensatz zu dem auf Perfusionsreaktoren beruhenden Ansatz handelsübliche Einwegzellkulturmaterialien verwenden, was die Anwendungsfreundlichkeit des erfindungsgemäßen Verfahrens und der erfindungsgemäßen Verwendung weiter steigert. Dies ist insbesondere bei der Durchführung von Testreihen mit entsprechender Anzahl an technischen Replikaten und Kontrollen von großer Bedeutung. Darüber hinaus kann die Geschwindigkeit der Trägerbewegung frei variiert werden, so dass die Bewegung gleichmäßig, in Intervallen oder schneller und langsamer werdend stattfinden kann.

Die Figuren zeigen:
Fig. 1 zeigt eine Bilderserie eines Trägers, welcher manuell in wässriger Lösung mit Hilfe von Dauermagneten bewegt wird.
Fig. 2 zeigt die Charakterisierung eines Trägers.
Fig. 3 zeigt das Ausmaß der Eisenfreisetzung und den Stoffaustausch in einem Träger.
Fig. 4 zeigt die Zellverträglichkeit eines Trägers sowie eines externen Magnetfeldes gegenüber humanen hämatopoetischen Stamm- und Progenitorzellen.
Fig. 5 zeigt den Einfluss eines Trägers sowie eines externen Magnetfeldes auf den Stammzellerhalt und die myeloide Differenzierung von humanen hämatopoetischen Stamm- und Progenitorzellen.
Fig. 6 zeigt schematisch das Arbeitsprinzip und eine photographische Aufnahme eines zur Trägerbewegung konstruierten Magnetlifts.
Fig. 7 zeigt die Beeinflussung des Stammzellerhalts bzw. der Differenzierung von humanen hämatopoetischen Stamm- und Progenitorzellen durch die Bewegung eines Trägers.

### Beispiele

Die nachstehenden Beispiele dienen der weiteren Erläuterung der vorliegenden Erfindung, ohne jedoch hierauf beschränkt zu sein.

Das erfindungsgemäße Verfahren zur dreidimensionalen Kultivierung von Zellen in einem flüssigen Nährmedium wurde anhand von humanen hämatopoetischen Stamm- und Progenitorzellen evaluiert.

### Herstellung und Charakterisierung des Trägers

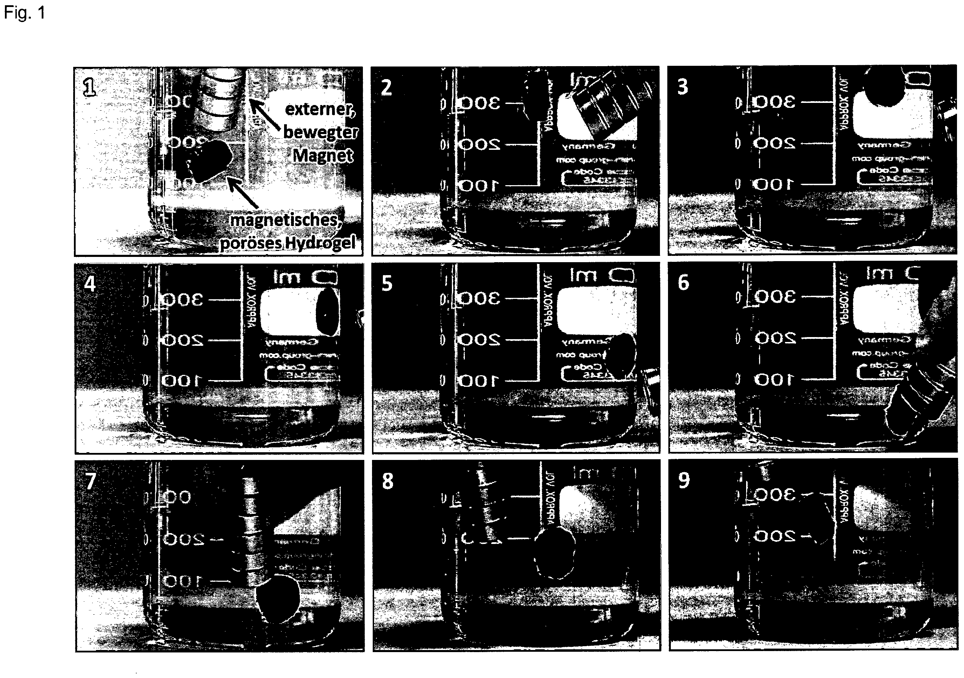

Als Träger wurden ein dreidimensionales, makroporöses Hydrogel mittels Salzauswaschen sowie ein flaches, nicht-poröses Hydrogel ohne Salzauswaschen unter Verwendung von Präpolymeren aus Poly(ethylenglykol)diacrylat (PEG-Diacrylat, PEGDA) hergestellt. Um magnetische Hydrogele (MH) zu erhalten, wurden der jeweiligen Präpolymerlösung funktionalisierte oder unfunktionalisierte, superparamagnetische Eisenoxidnanopartikel aus Magnetit hinzugefügt. Neben den magnetischen Hydrogelen wurden auch solche ohne magnetische Eigenschaften hergestellt. Die so erhaltenen Hydrogele wurden in einem mit einer wässrigen Lösung gefüllten Kulturgefäß bereitgestellt. In diesem konnten die Hydrogele mit Hilfe von sich außerhalb des Gefäßes befindlichen Dauermagneten bewegt werden (Fig. 1). Im Einzelnen erfolgten die Funktionalisierung der magnetischen Nanopartikel (MP) sowie die Herstellung der magnetischen und nicht-magnetischen Hydrogele wie folgt:

### Funktionalisierung der magnetischen Nanopartikel mit Methacrylateinheiten

1 g der magnetischen Nanopartikel (Chemagen) mit einem gemittelten Teilchendurchmesser von 10 nm wurde in 2 mL einer 33 %-igen (*V*/*V*) ethanolischen Lösung dispergiert. Die so erhaltene Dispersion wurde bei Raumtemperatur auf einem Kreisschüttler geschüttelt. Daraufhin wurde 1 mL 3-(Trimethoxysilyl)propylmethacrylat (MEMO; Sigma-Aldrich) in Abständen von 20 Minuten und in Inkrementen von 200 µL hinzugetropft. Die so funktionalisierten magnetischen Nanopartikel wurden fünfmal gewaschen, bevor sie weiter verwendet wurden.

### Herstellung des flachen, nicht-porösen PEG-Hydrogels

1 mL einer 33 %-igen (*w*/*V*) PEGDA-Lösung wurde durch Rühren mit einem Magnetrührer bei 130 rpm für eine Dauer von 30 Minuten erhalten. Anschließend wurden 40 µL einer RGD-Acrylat-Stammlösung mit einer Konzentration von 500 µM hinzugegeben, um eine Endkonzentration von 20 µM RGD in der Lösung des Hydrogelpräkursors zu erhalten, wobei für weitere 10 Minuten gerührt wurde. Ferner wurden 30 µL einer 10 %-igen (*w*/*V*) Ammoniumpersulfat-Lösung (APS; AppliChem) zu 500 µL der Lösung des Hydrogelpräkursors hinzugefügt und mit einem Spatel vermischt. Daraufhin wurden 5 µL *N,N,N,N*-Tetramethylethylendiamin (TEMED; Merck) hinzugegeben und rasch mit einem Spatel vermischt. Die so erhaltene Lösung wurde unmittelbar zwischen zwei im Abstand von 250 µm befindlichen Glasträgern pipettiert. Nach 30 Minuten wurde das so gebildete Hydrogel über Nacht in Wasser inkubiert, um es aufquellen zu lassen.

Zur Herstellung des flachen, nicht-porösen PEG-Hydrogels mit magnetischen Eigenschaften wurden 60 mg der unfunktionalisierten magnetischen Nanopartikel in 1 mL Wasser überführt. Hierzu wurden die vorstehend beschriebenen Edukte zur Herstellung des Hydrogels gegeben und mit einem Spatel unter Rühren vermischt. Die Herstellung des magnetischen Hydrogels erfolgte ansonsten analog zur Herstellung des nicht-magnetischen Hydrogels.

### Herstellung des dreidimensionalen, makroporösen PEG-Hydrogels

Im Stand der Technik ist die Herstellung von dreidimensionalen, makroporösen PEG-Hydrogelen eingehend beschrieben (Raic & Rödling et al., 2014; Rödling et al., 2014). Im vorliegenden Fall wurde eine 33 %-ige (*w*/*V*) PEGDA-Lösung, gesättigt mit Natriumchlorid (VWR chemicals), hergestellt und es wurde RGD-Acrylat bis zu einer Endkonzentration von 20 µM hinzugegeben.

150 mg der so erhaltenen Lösung des Hydrogelpräkursors wurden zu 600 mg NaCl-Kristallen mit einer Größe im Bereich von 40 bis 100 µm hinzugegeben, wobei die NaCI-Kristalle als Porogen dienten. Das Gemisch wurde in eine 48-Wellplate (Greiner Bio-One) überführt und das PEGDA wurde durch Hinzufügen von 54 µL APS mit einer Konzentration von 10 % (*w*/*V*) sowie 8 µL TEMED vernetzt. Nach 30 Minuten wurde das so erhaltene Hydrogel in 500 mL Wasser für eine Dauer von drei Tagen inkubiert, um die eingeschlossenen NaCI-Kristalle auszuwaschen. Das Wasser wurde zu diesem Zweck mehrere Male ausgetauscht.

Zur Herstellung des dreidimensionalen, makroporösen PEG-Hydrogels mit magnetischen Eigenschaften wurden 60 mg der magnetischen Nanopartikel, entweder unfunktionalisiert zur nicht-kovalenten Anbindung oder mit Methacrylateinheiten funktionalisiert zur kovalenten Anbindung, in 1 mL einer gesättigten NaCI-Lösung überführt. Hierzu wurden die vorstehend beschriebenen Edukte zur Herstellung des Hydrogels gegeben und mit einem Spatel unter Rühren vermischt. Die Herstellung des magnetischen Hydrogels erfolgte ansonsten analog zur Herstellung des nicht-magnetischen Hydrogels.

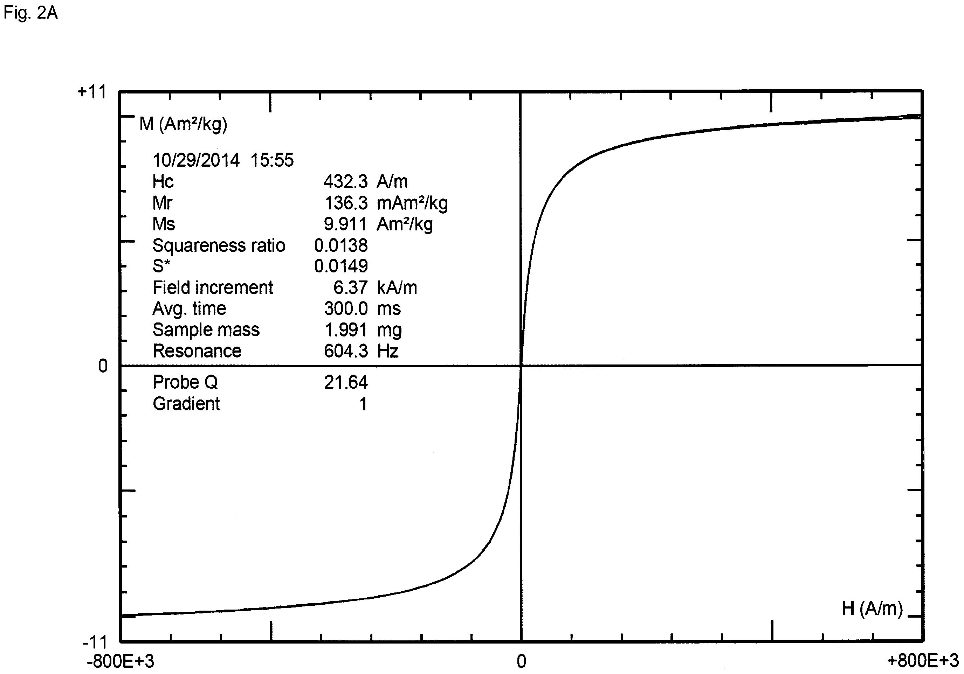

Die magnetischen Eigenschaften der Hydrogele wurden mit Hilfe eines Magnetometers (Typ: Alternating Gradient Magnetometer, AGM) charakterisiert (Fig. 2A). Auf der Ordinate ist die Magnetisierung *M* in A·m²/kg und auf der Abszisse ist die magnetische Feldstärke *H* in A/m aufgetragen.

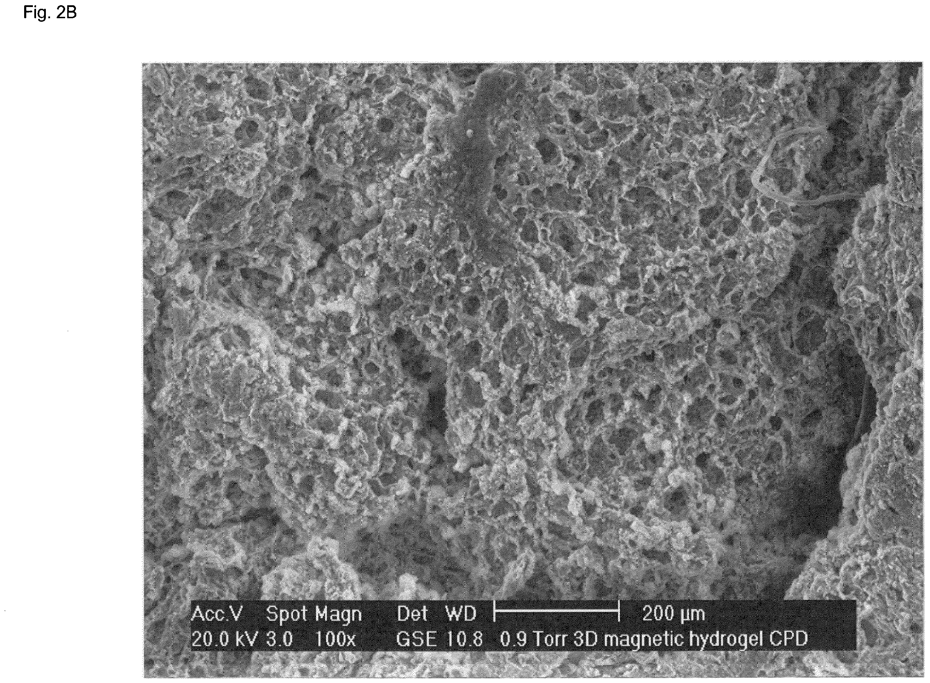

Die makroporösen Eigenschaften der Hydrogele wurden mit Hilfe rasterelektronenmikroskopischer Messungen untersucht (Fig. 2B). Der Maßstabsbalken in der repräsentativen rasterelektronenmikroskopischen Aufnahme entspricht einer Abmessung von 200 µm.

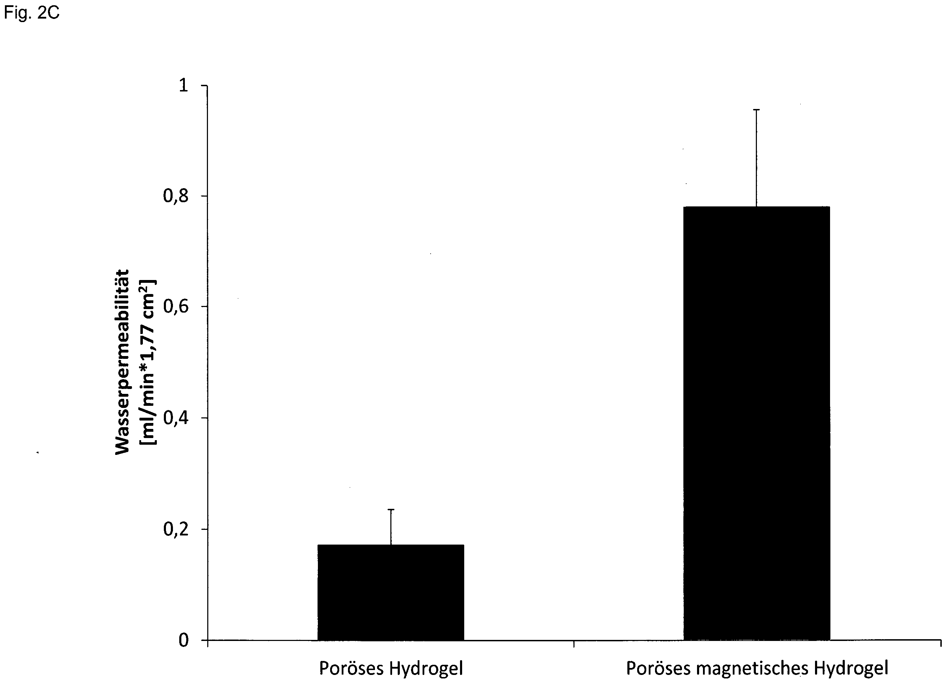

Die Wasserpermeabilität der Hydrogele wurde mit Hilfe von Wassersäulen überprüft (Fig. 2C). Durch Installation von Wassersäulen definierter Höhe über den Hydrogelen mit und ohne magnetische(n) Nanopartikel(n) wurde der Durchlass an Wasservolumen pro Zeitintervall ermittelt.

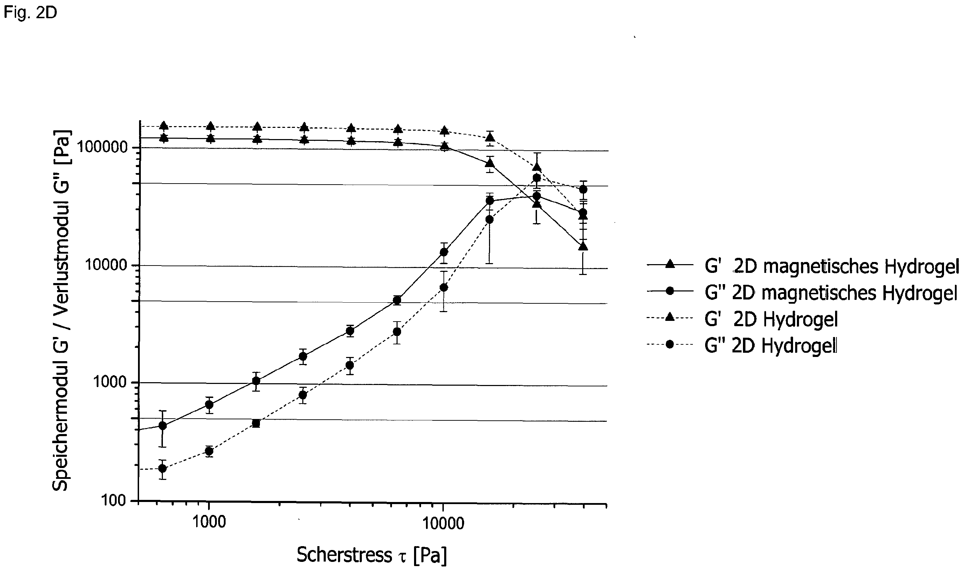

Die mechanischen Eigenschaften der Hydrogele wurden rheometrisch bestimmt (Fig. 2D). Dargestellt sind der Speichermodulus (*G*') sowie der Verlustmodulus (*G*") der Hydrogele mit und ohne magnetische(n) Nanopartikel(n).

### Stabilität des Trägers

Um Aussagen über die Stabilität des Trägers zu erhalten, wurden die magnetischen Nanopartikel entweder ohne vorherige Funktionalisierung nicht-kovalent in das Hydrogel eingebunden oder nach Funktionalisierung mit Methacrylatgruppen während der Polymerisation kovalent in das Hydrogel einpolymerisiert. Die aus den Hydrogelen freigesetzte Menge an Eisen, welche von den magnetischen Nanopartikeln herrührt, wurde emissionsspektrometrisch (Typ: optische Emissionsspektrometrie mit induktiv gekoppeltem Plasma, ICP-OES) anhand des jeweiligen Überstandes an verschiedenen Zeitpunkten über einen Zeitraum von insgesamt 35 Tagen bestimmt.

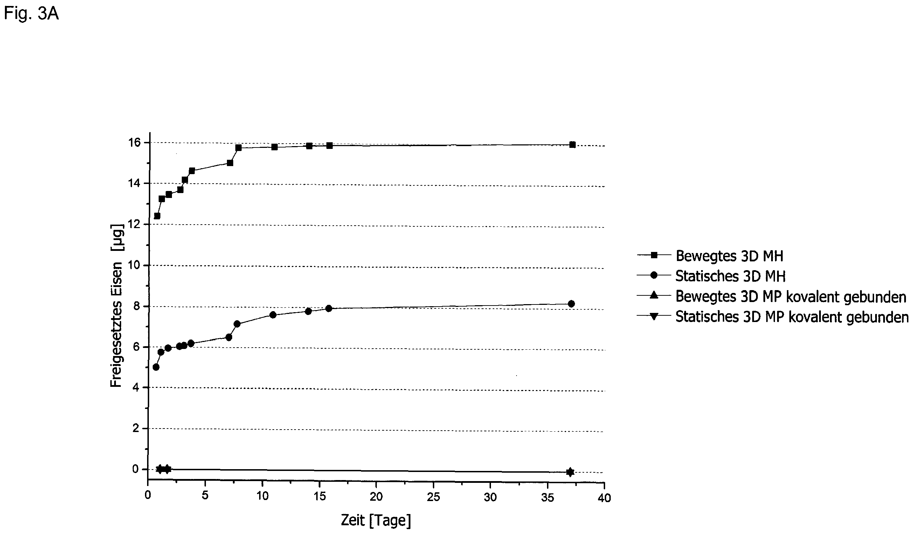

Im Falle der nicht-funktionalisierten magnetischen Nanopartikel war die Eisenfreisetzung bei Bewegung der Hydrogele in wässriger Lösung ausgeprägter als bei unbewegter, d.h. statischer Inkubation. Nach Funktionalisierung der magnetischen Nanopartikel mit Methacrylatgruppen konnten diese kovalent in die Matrix des Hydrogels eingebunden werden, was zu einer erhöhten Stabilität der magnetischen Nanopartikel in dem Hydrogel führte. Dies zeigte sich anhand der äußerst geringen Eisenfreisetzung sowohl bei bewegter als auch bei unbewegter Inkubation der Hydrogele in wässriger Lösung (Fig. 3A).

### Stoffaustausch des Trägers

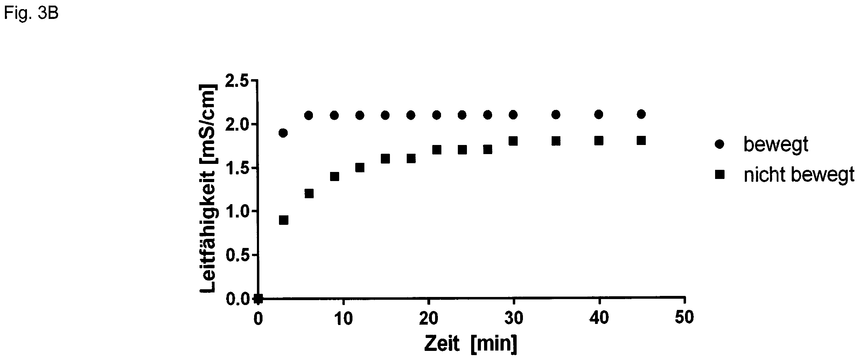

Zur Beurteilung der Frage, ob die Bewegung des Trägers in wässriger Lösung zu einem erhöhten Stoffaustausch führt, wurde das Hydrogel zunächst in einer NaCl-Lösung inkubiert, mit der es sich vollsaugte, und anschließend in destilliertem Wasser unbewegt oder bewegt gehalten. Anhand von Leitfähigkeitsmessungen in der das Hydrogel umgebenden wässrigen Lösung konnte auf die Freisetzung der Ionen aus dem zuvor in NaCI-Lösung inkubierten Hydrogel in das destillierte Wasser geschlossen werden. Die Ergebnisse zeigen, dass die Bewegung des Hydrogels zu einer beschleunigten Freisetzung der Ionen und damit zu einem erhöhten Stoffaustausch mit der wässrigen Lösung führte (Fig. 3B).

### Zellverträglichkeit des Trägers und des externen Magnetfeldes

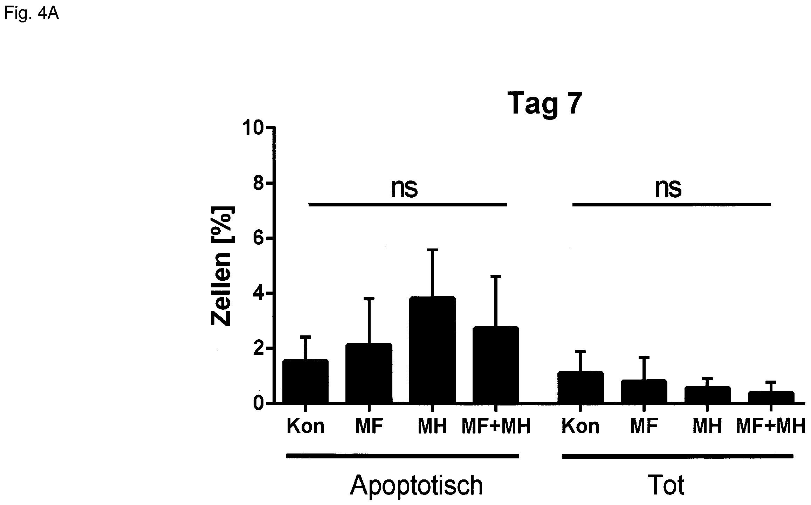

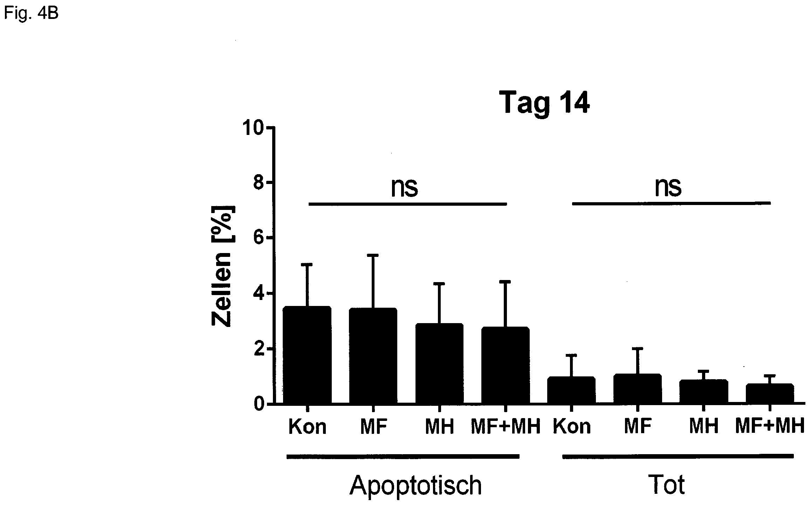

Die Zellverträglichkeit des Trägers wie auch des externen Magnetfeldes wurde mit Hilfe von Apoptosetests, Proliferations- und Differenzierungsstudien nachgewiesen. Die untersuchten humanen hämatopoetischen Stamm- und Progenitorzellen zeigten in den Apoptosetests keine erhöhte Zellsterblichkeit bei Anwesenheit der magnetischen Hydrogele (MH), des externen Magnetfeldes (MF) oder von beiden (MF+MH) im Vergleich zur Kontrolle (Kon), welche eine hydrogelfreie Standardkultivierung auf Zellkulturplastik ohne magnetische Nanopartikel und bei Abwesenheit des externen Magnetfeldes darstellte (Fig. 4A und 4B).

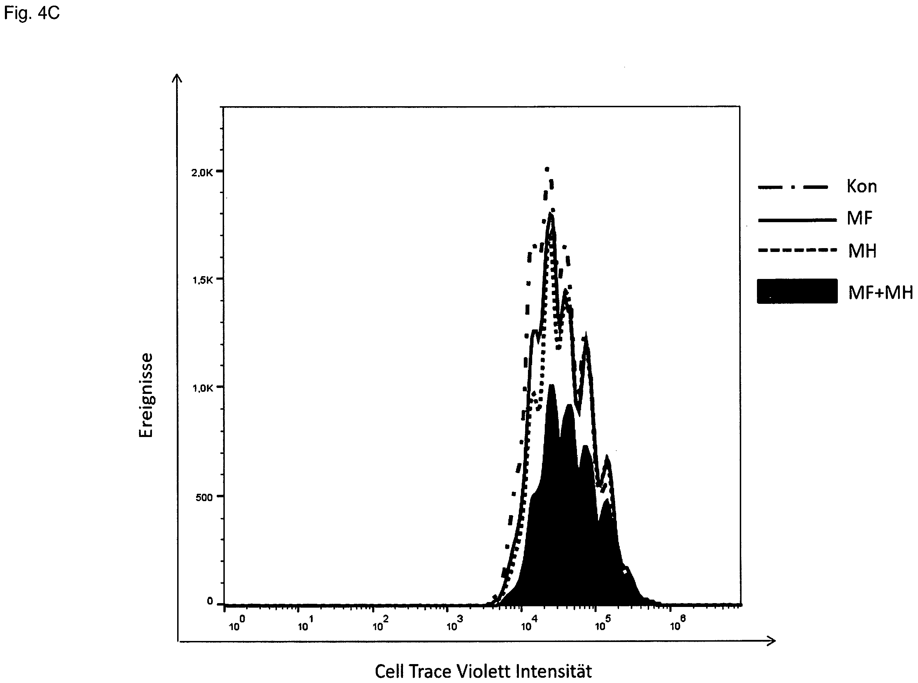

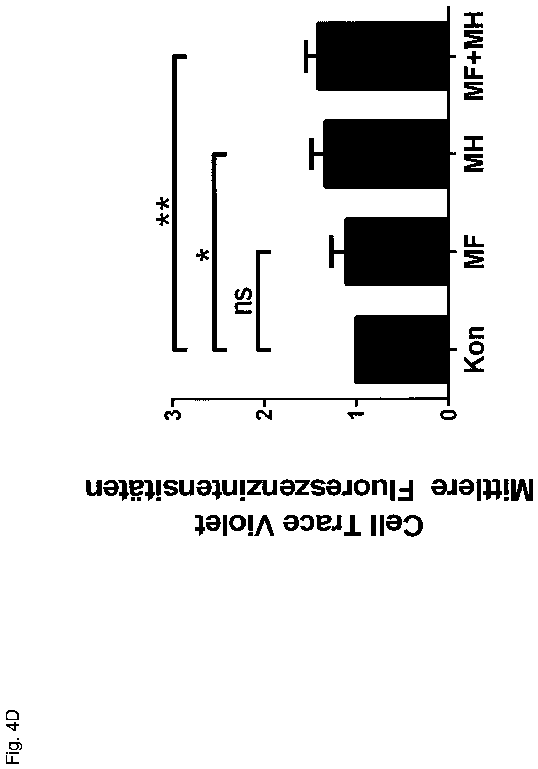

Auch die Proliferation, untersucht mittels Cell Trace Violet-Färbung der Zellen, und die Entwicklung der Zellzahl über die Zeit wurden durch die magnetischen Eigenschaften der Hydrogele und/oder durch das externe Magnetfeld nicht signifikant beeinflusst. Cell Trace Violet ist ein Farbstoff, der nach der Anfärbung der Zellen in deren Zytoplasma detektierbar ist und sich bei jeder Zellteilung gleichmäßig auf die zwei Tochterzellen verteilt, so dass sich die Intensität der Färbung halbiert. Durch Analyse der Fluoreszenzintensität der gefärbten Zellen lässt sich auf die Zahl der durchlaufenen Zellteilungen und damit auf das Ausmaß der Proliferation schließen. Die mittlere Fluoreszenzintensität des Cell Trace Violet-Signals wurde hier als ein Maß für das Ausmaß der Proliferation der Zellen im Beobachtungszeitraum herangezogen (Fig. 4C und 4D).

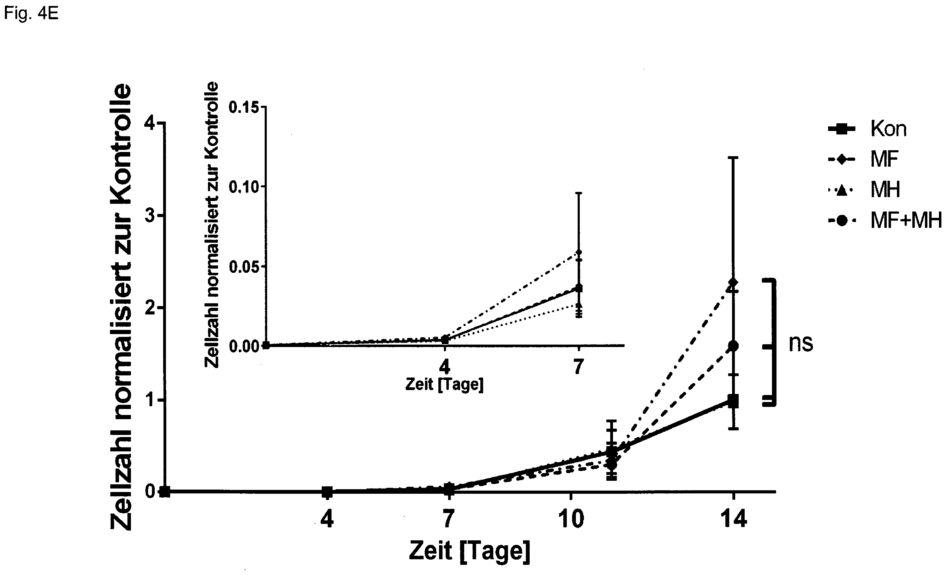

Die Zellzahl war von den verschiedenen Bedingungen über einen Zeitraum von 14 Tagen unbeeinflusst (Fig. 4E). Die Ordinate zeigt die normalisierte Zellzahl und die Abszisse zeigt die Kulturzeit in Tagen.

### Einfluss des Trägers und des externen Magnetfeldes auf den Stammzellerhalt und die myeloide Differenzierung

Der Erhalt des Stammzellcharakters von hämatopoetischen Stamm- und Progenitorzellen wurde über den Stammzellmarker CD34 verfolgt und die myeloide Differenzierung wurde über einen sogenannten Koloniebildungsassay (colony forming assay) untersucht, der die retrospektive Analyse der Zahl und Art myeloider Progenitorzellen in der Kultur erlaubt. In Bezug auf die Zahl und den Prozentsatz der Zellen, die nach 7 und 14 Tagen in Kultur noch den Stammzellmarker CD34 aufwiesen, konnte kein Einfluss der magnetischen Hydrogele, des externen Magnetfeldes oder von beiden festgestellt werden. Beim Koloniebildungsassay zeigte sich, dass die magnetischen Nanopartikel in den Hydrogelen einen positiven Einfluss auf die Bildung von Vorläuferzellen hatten, aus denen Granulozyten, Monozyten und Makrophagen hervorgehen können.

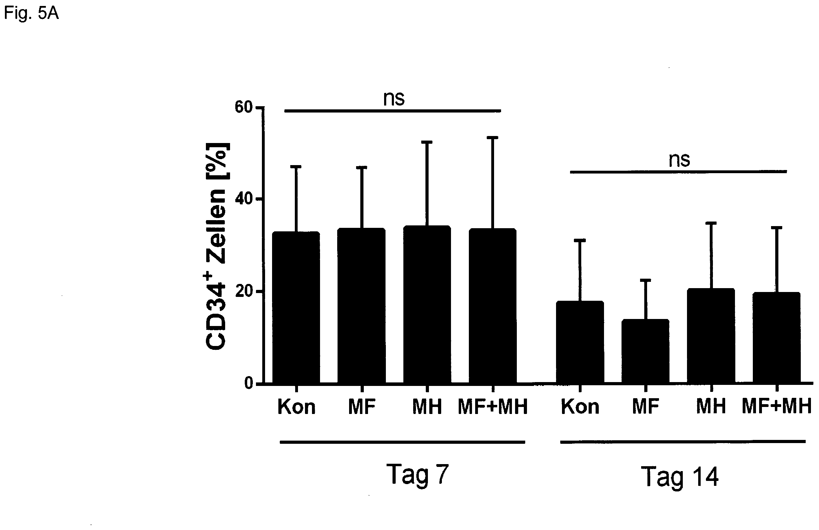

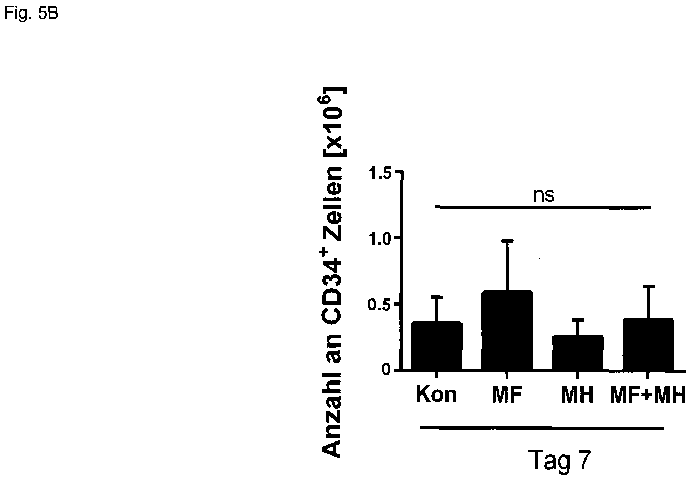

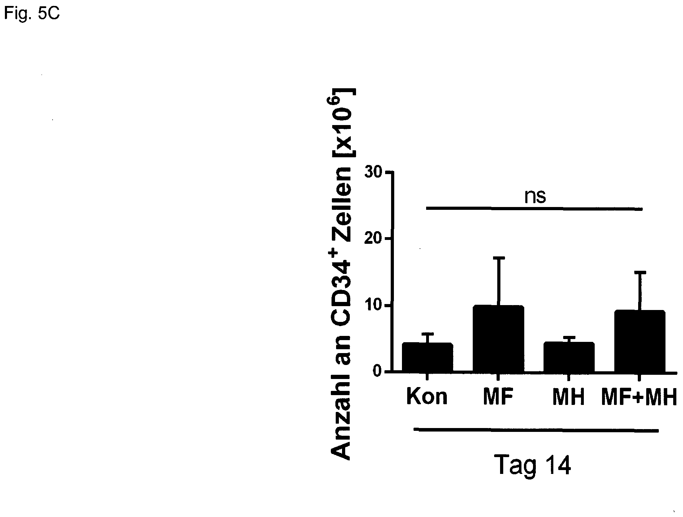

Die detaillierten Ergebnisse sind nachstehend aufgeführt:
Der Prozentsatz an Zellen, die den Stammzellmarker CD34 exprimierten (CD34⁺), wurde mittels Durchflusszytometrie ermittelt. Weder das externe Magnetfeld (MF) noch die magnetischen Hydrogele (MH) oder eine Kombination hiervon (MF+MH) zeigte einen Einfluss auf den Prozentsatz an CD34⁺-Zellen nach 7 und 14 Tagen (Fig. 5A). Ebenso war die Zahl der gefundenen CD34⁺-Zellen unter allen Bedingungen nach 7 Tagen (Fig. 5B) und nach 14 Tagen (Fig. 5C) gleich groß.

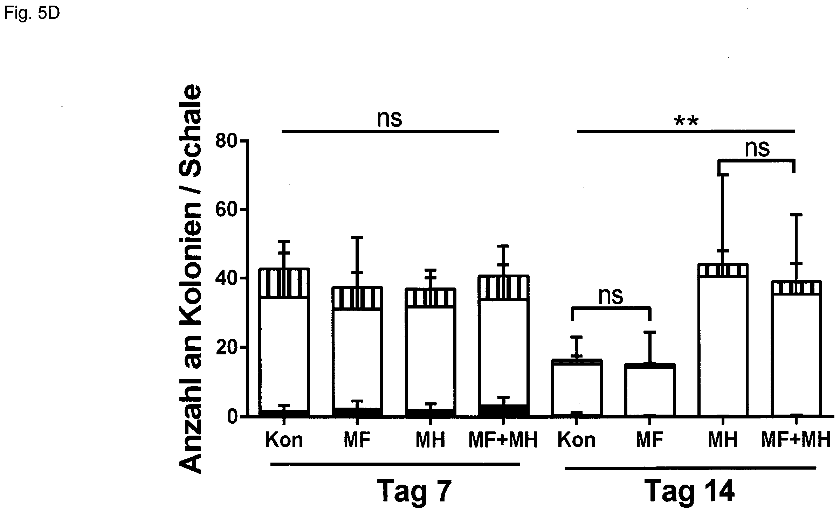

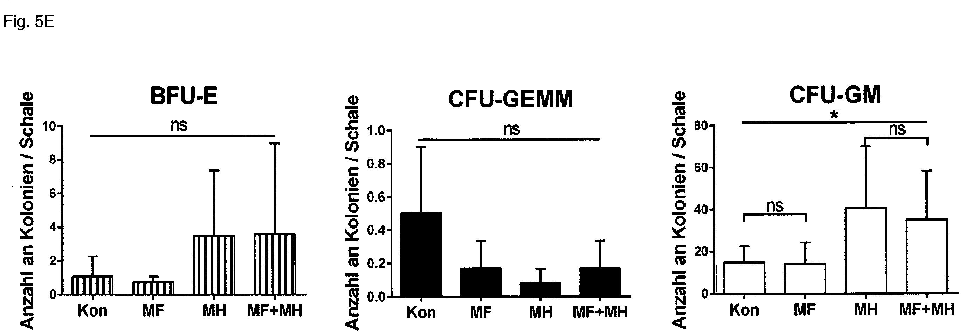

Das myeloide Differenzierungspotential, welches über die Zahl gebildeter myeloider Kolonien gemessen wurde, war nach 7 Tagen unbeeinflusst. Nach 14 Tagen hatten die magnetischen Hydrogele in Gegenwart und Abwesenheit des externen Magnetfeldes einen positiven Einfluss auf die myeloide Differenzierung (Fig. 5D und 5E). Im Detail waren myeloide Progenitorzellen, die in Granulozyten, Monozyten und Makrophagen differenzieren können, von diesem positiven Effekt betroffen.

### Anwendung des Trägers in einem Verfahren zur dreidimensionalen Kultivierung von Zellen

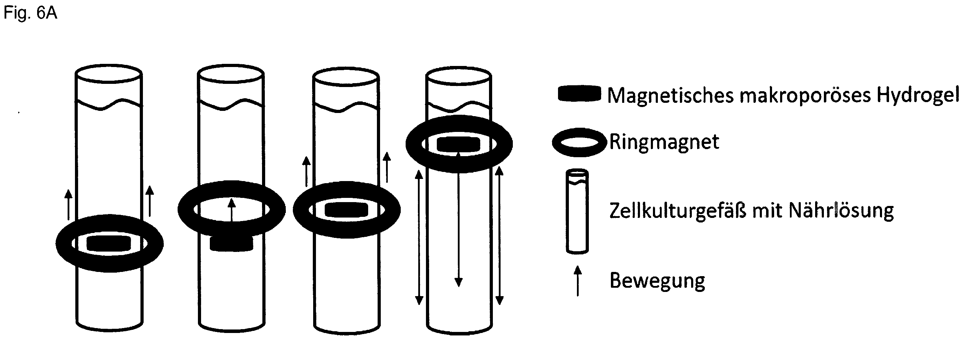

Zur dreidimensionalen Kultivierung von Zellen unter Bewegung des Trägers in einem flüssigen Nährmedium wurde ein Magnetlift konstruiert. Dieser erlaubte es einen Ringmagneten, welcher ein Kulturgefäß umschloss, zu positionieren und zu bewegen. Das Magnetfeld, das in der inneren Öffnung des Ringmagneten herrschte, positionierte den magnetischen Träger im Inneren des Kulturgefäßes an der vertikalen Position des Ringmagneten. Durch die Bewegung des Ringmagneten konnte der Träger in der wässrigen Lösung im Inneren des Kulturgefäßes auf und ab bewegt werden. Auf diese Weise war eine berührungsfreie Bewegung des Trägers durch das flüssige Nährmedium möglich, wodurch der Stoffaustausch begünstigt wurde (Fig. 6A).

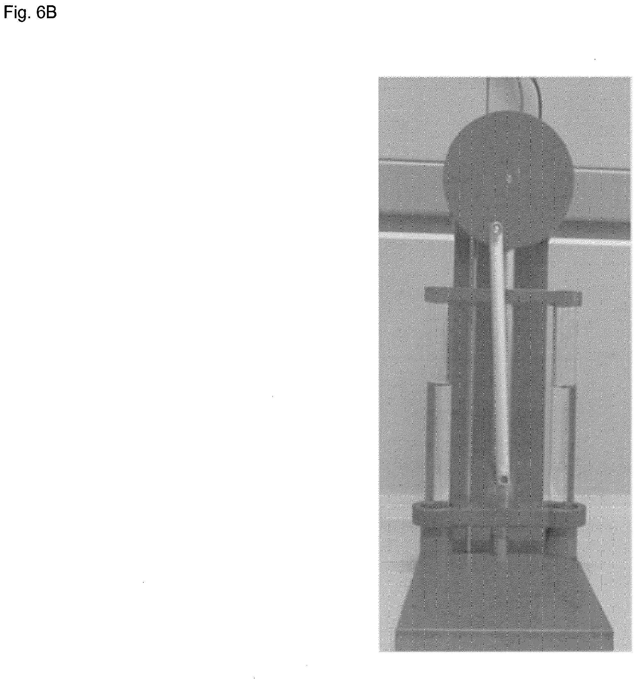

Bei dem hier beschriebenen Magnetlift trieb ein Motor eine Kurbelscheibe an, über die eine Pleuelstange bewegt wurde, an deren unteren Ende eine Vorrichtung mit zwei Ringmagneten angebracht war (Fig. 6B). Die Apparatur wurde zur Kultivierung der Zellen im Inneren eines CO₂-lnkubators betrieben, welcher das Einstellen von Standardzellkulturbedingungen (37 °C, 5 % CO₂, gesättigte Luftfeuchtigkeit) erlaubte.

### Beeinflussung des Stammzellerhalts bzw. der Differenzierung durch die Bewegung des Trägers

Die in den vorliegenden Beispielen verwendeten humanen hämatopoetischen Stamm- und Progenitorzellen sind die einzige Sorte von Stammzellen, welche routinemäßig zur Behandlung von Patienten, insbesondere von Leukämiepatienten, eingesetzt werden. Die Entwicklung von Möglichkeiten zur kontrollierten Proliferation oder Differenzierung dieser Zellen im Labor würde die Behandlung einer weitaus größeren Anzahl an Patienten ermöglichen und hätte weitreichende positive Effekte für die Behandlung vieler Patienten. Hämatopoetische Stamm- und Progenitorzellen kommen natürlicherweise im Knochenmark vor, wo sie durch die dreidimensionale Architektur ihrer Umgebung, die in der schwammartig strukturierten Spongiosa des Knochens liegt, durch die Wechselwirkungen mit der extrazellulären Matrix und mit anderen Zellen in ihrer Nachbarschaft sowie durch lösliche Faktoren in ihrem Verhalten kontrolliert werden.

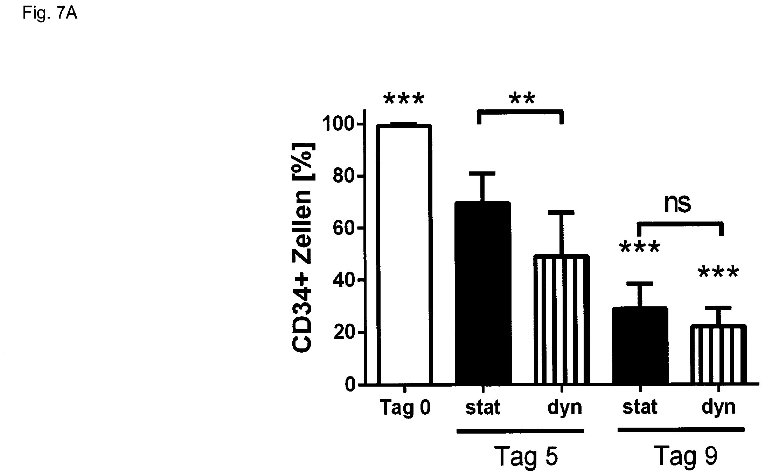

Im vorliegenden Fall wurde die dreidimensionale Architektur der Spongiosa des Knochens durch das Hydrogel imitiert, und die adhäsiven Eigenschaften der extrazellulären Matrix wurden durch ein einpolymerisiertes adhäsives Peptid mit RGD-Sequenz simuliert. Als Zellen aus der Umgebung der humanen hämatopoetischen Stamm- und Progenitorzellen im Knochenmark wurden mesenchymale Stamm-/Stromazellen eingesetzt. Lösliche Faktoren wurden über das flüssige Nährmedium zugesetzt. Es zeigte sich, dass es während der ersten 5 Tage in den über den Magnetlift bewegten Hydrogelen zu einer erhöhten Differenzierung der Zellen kam, was sich in einem schnelleren Verlust des Stammzellmarkers CD34 zeigte. Der Prozentsatz an Zellen, die den Stammzellmarker CD34, der während der fortschreitenden Differenzierung von hämatopoetischen Zellen verloren geht, auf ihrer Oberfläche trugen (CD34⁺-Zellen; auf der Ordinate aufgetragen), war nach 5 Tagen geringer, wenn die Hydrogele, in denen die Zellen kultiviert wurden, über den Magnetlift in dem flüssigen Nährmedium bewegt wurden (dynamische Kultur; dyn) als wenn die Träger mit den Zellen unbewegt inkubiert wurden (statische Kultur; stat) (Fig. 7A). Zu späteren Zeitpunkten war dieser Effekt nicht mehr zu beobachten. Dies bedeutet, dass durch die Bewegung des Trägers das Verhalten der humanen hämatopoetischen Stamm- und Progenitorzellen hinsichtlich Erhalt des Stammzellcharakters und Differenzierung beeinflusst werden konnte und somit als ein einfach zu kontrollierender Parameter zur Steuerung des Verhaltens von Stammzellen zur Verfügung steht.

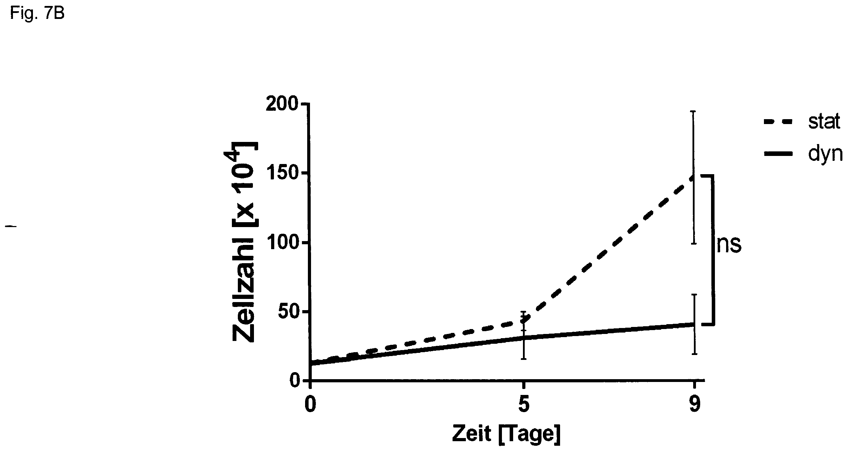

Die Gesamtzellzahl war über den Beobachtungszeitraum unabhängig davon, ob der Träger in der Nährlösung über den Magnetlift bewegt wurde (dyn) oder unbewegt inkubiert wurde (stat) (Fig. 7B).

## Patentansprüche

1. Verfahren zur dreidimensionalen Kultivierung von Zellen in einem flüssigen Nährmedium, wobei sich die Zellen in den Poren eines Trägers mit magnetischen Eigenschaften befinden, **dadurch gekennzeichnet, dass**
der Träger durch ein externes Magnetfeld innerhalb des flüssigen Nährmediums bewegt wird und der Träger ein makroporöses Polymernetzwerk und magnetische Nanopartikel, welche kovalent oder nicht-kovalent an das Polymernetzwerk gebunden sind, umfasst, wobei die Porenräume des Trägers in offenem Kontakt zueinander stehen, so dass ein Stoffaustausch mit dem flüssigen Nährmedium stattfindet.

2. Verfahren nach Anspruch 1, wobei die Bewegung und Positionierung des Trägers berührungsfrei erfolgen.

3. Verfahren nach Anspruch 1 oder 2, wobei das externe Magnetfeld durch einen sich außerhalb des flüssigen Nährmediums befindlichen Magneten hervorgerufen wird.

4. Verfahren nach Anspruch 3, wobei der Magnet ein das flüssige Nährmedium umgebender Ringmagnet ist.

5. Verfahren nach Anspruch 3 oder 4, wobei das externe Magnetfeld durch Positionsänderung des Magneten in Bezug auf das flüssige Nährmedium räumlich variiert wird.

6. Verfahren nach Anspruch 1 oder 2, wobei das externe Magnetfeld elektromagnetisch hervorgerufen wird.

7. Verfahren nach Anspruch 6, wobei das externe Magnetfeld durch eine zeitlich variierende Stromstärke in einer Anordnung von mindestens zwei Elektromagnetspulen räumlich variiert wird.

8. Verfahren nach Anspruch 5 oder 7, wobei durch räumliche Variation des externen Magnetfeldes der Träger entlang einer vorgegebenen Raumrichtung hin und her bewegt wird.

9. Verfahren nach einem der Ansprüche 1 bis 8, wobei das Polymernetzwerk Einheiten aus Poly(ethylenglykol) umfasst.

10. Verfahren nach einem der Ansprüche 1 bis 9, wobei das Polymernetzwerk weiter funktionalisiert ist.

11. Verwendung eines Trägers mit magnetischen Eigenschaften zur dreidimensionalen Kultivierung von Zellen in einem flüssigen Nährmedium, wobei der Träger Poren aufweist, die zur Aufnahme von Zellen geeignet sind, **dadurch gekennzeichnet, dass**
der Träger durch ein externes Magnetfeld innerhalb des flüssigen Nährmediums bewegt wird und der Träger ein makroporöses Polymernetzwerk und magnetische Nanopartikel, welche kovalent oder nicht-kovalent an das Polymernetzwerk gebunden sind, umfasst, wobei die Porenräume des Trägers in offenem Kontakt zueinander stehen, so dass ein Stoffaustausch mit dem flüssigen Nährmedium stattfindet.

## Claims

1. A method for three-dimensional culturing of cells in a liquid nutrient medium, wherein the cells are located in the pores of a carrier having magnetic properties, **characterized in that**
the carrier is moved by an external magnetic field within the liquid nutrient medium, and the carrier comprises a macroporous polymer network and magnetic nanoparticles which are bonded to the polymer network covalently or non-covalently, wherein the pore volumes of the carrier are in open contact with one another so that material exchange takes place with the liquid nutrient medium.

2. The method according to claim 1, wherein the movement and positioning of the carrier are conducted in a non-contact manner.

3. The method according to claim 1 or 2, wherein the external magnetic field is caused by a magnet located outside the liquid nutrient medium.

4. The method according to claim 3, wherein the magnet is a ring magnet surrounding the liquid nutrient medium.

5. The method according to claim 3 or 4, wherein the external magnetic field is spatially varied by changing the position of the magnet in relation to the liquid nutrient medium.

6. The method according to claim 1 or 2, wherein the external magnetic field is caused electromagnetically.

7. The method according to claim 6, wherein the external magnetic field is spatially varied by a current varying over time in an arrangement of at least two electromagnetic coils.

8. The method according to claim 5 or 7, wherein the carrier is moved back and forth along a predetermined spatial direction by spatial variation of the external magnetic field.

9. The method according to any one of claims 1 to 8, wherein the polymer network comprises units of poly(ethylene glycol).

10. The method according to any one of claims 1 to 9, wherein the polymer network is further functionalized.

11. Use of a carrier having magnetic properties for three-dimensional culturing of cells in a liquid nutrient medium, wherein the carrier has pores suitable for receiving cells, **characterized in that**
the carrier is moved by an external magnetic field within the liquid nutrient medium, and the carrier comprises a macroporous polymer network and magnetic nanoparticles which are bonded to the polymer network covalently or non-covalently, wherein the pore volumes of the carrier are in open contact with one another so that material exchange takes place with the liquid nutrient medium.

## Revendications

1. Procédé de culture tridimensionnelle de cellules dans un milieu de culture liquide, dans lequel les cellules se trouvent dans les pores d'un support présentant des propriétés magnétiques, **caractérisé en ce que**
le support est agité par un champ magnétique externe à l'intérieur du milieu de culture liquide et le support comprend un réseau polymère macroporeux et des nanoparticules magnétiques, lesquelles sont liées au réseau polymère de manière covalente ou non covalente, les espaces des pores du support étant en contact ouvert l'un par rapport à l'autre de sorte qu'il se produit un échange de substance avec le milieu de culture liquide.

2. Procédé selon la revendication 1, dans lequel l'agitation et le positionnement du support se font sans contact.

3. Procédé selon la revendication 1 ou 2, dans lequel le champ magnétique externe est causé par un aimant se trouvant à l'extérieur du milieu de culture liquide.

4. Procédé selon la revendication 3, dans lequel l'aimant est un aimant circulaire entourant le milieu de culture liquide.

5. Procédé selon la revendication 3 ou 4, dans lequel le champ magnétique externe varie dans l'espace par un changement de position de l'aimant par rapport au milieu de culture liquide.

6. Procédé selon la revendication 1 ou 2, dans lequel le champ magnétique externe est causé de manière électromagnétique.

7. Procédé selon la revendication 6, dans lequel le champ magnétique externe varie dans l'espace par variation temporelle de l'intensité électrique dans un agencement d'au moins deux bobines électromagnétiques.

8. Procédé selon la revendication 5 ou 7, dans lequel par variation spatiale du champ magnétique externe, le support est agité en va-et-vient sur une direction spatiale prédéfinie.

9. Procédé selon l'une des revendications 1 à 8, dans lequel le réseau polymère comprend des motifs de poly(éthylèneglycol).

10. Procédé selon l'une des revendications 1 à 9, dans lequel le réseau polymère est en outre fonctionnalisé.

11. Utilisation d'un support présentant des propriétés magnétiques destinée à la culture tridimensionnelle de cellules dans un milieu de culture liquide, le support comprenant des pores convenant à l'absorption de cellules, **caractérisée en ce que** le support est agité par un champ magnétique externe à l'intérieur du milieu de culture liquide et le support comprend un réseau polymère macroporeux et des nanoparticules magnétiques, lesquelles sont liées au réseau polymère de manière covalente ou non covalente, les espaces des pores du support étant en contact ouvert l'un par rapport à l'autre de sorte qu'il se produit un échange de substance avec le milieu de culture liquide.
